# EUROPEAN PATENT SPECIFICATION

(11) **EP 2 480 133 B1**
(45) Date of publication and mention of the grant of the patent: **12.08.2015**
(21) Application number: 10760093.4
(22) Date of filing: 16.09.2010
(51) Int. Cl.: A61B 5/00, A61B 6/00, G01R 33/54

(54) **APPARATUS AND METHOD FOR ACQUIRING DIAGNOSTIC INFORMATION**
VORRICHTUNG UND VERFAHREN ZUR ERFASSUNG VON DIAGNOSTISCHEN FORMATIONEN
APPAREIL ET PROCÉDÉ POUR L'ACQUISITION D'INFORMATIONS DE DIAGNOSTIC

(30) Priority: 22.09.2009 EP 09170899
(43) Date of publication of application: 01.08.2012
(73) Proprietor: Koninklijke Philips N.V., 5656 AE Eindhoven (NL)
(72) Inventor: HOLTHUIZEN, Ronaldus, Frederik, Johannes, NL-5656 AE Eindhoven (NL); DEN HARDER, Johan, Michiel, NL-5656 AE Eindhoven (NL); DE KOK, Wendy, NL-5656 AE Eindhoven (NL)
(74) Representative: van Velzen, Maaike Mathilde
(86) International application number: PCT/IB2010/054185
(87) International publication number: WO 2011/036613

(56) References cited:
- US-A1- 2002 156 359
- US-A1- 2006 072 700
- US-A1- 2006 173 268
- US-A1- 2006 193 437
- US-A1- 2007 055 135

## Description

### FIELD OF THE INVENTION

The invention relates to an apparatus for acquiring diagnostic information comprising:
- a data acquisition module for acquiring image data of at least a part of a person's anatomy,
- a planning module defining with reference to a spatial position and orientation of an example anatomy at least one series of scanning steps to be executed with the data acquisition module on an actual anatomy,
- a user interface to adjust imaging parameters to be used in a selected scanning step of a series of scanning steps, for acquiring image data of that actual anatomy.

### BACKGROUND OF THE INVENTION

Such an apparatus is known from WO2006/013499. In this known apparatus a fully automatic calculation and determination of the scanning parameters that will pertain to the imaging of a person's actual anatomy is carried out, which is based on default parameters that pertain to a scanning step concerning the anatomy. The parameters that are automatically calculated describe a set of slices to be acquired during the operation of the acquisition module on the actual anatomy. The user interface of the known apparatus is only used to allow the user -if desired- to modify the setting of the parameters that are automatically generated for the acquisition of the image data concerning the actual anatomy.

In this invention, similarly as in WO2006/013499, the data acquisition module can be any conceivable instrument, such as a magnetic resonance unit, a computer tomography unit, or any other type of instrument that is commonly used for the acquisition of image data of an anatomy. Although the invention is generally applicable for the acquisition of image data of any particular anatomy or part thereof, it is best explained with reference to the acquisition of cardiac image data when using a magnetic resonance unit. In view thereof in the following a discussion will be offered concerning the acquisition of cardiac image data with MRI, albeit that it is explicitly remarked that the invention is not restricted to this particular application.

Planning of cardiac examinations is perceived to be difficult for (novice) users in particular when using a magnetic resonance unit. In MRI considered planning of the slices to be imaged is required in order to combat the time that is needed for data acquisition. This remains a difficult task, also for more experienced users, due to the fact that this type of imaging does not occur frequently. There is therefore a need for an apparatus and method which offers a user-friendly possibility to plan the examination and the scanning steps to be carried out in the imaging of a particular anatomy.

In US patent application 2002/0156359 A1, a medical imaging system is described for generating views of a heart along anatomically useful planes. A method for generating the views includes generating a prescription image of a heart, receiving data regarding a position of at least one landmark on the prescription image to define a series of slices through the heart along at least one anatomical plane, generating a plurality of images under software control, and saving the generated images. The medical imaging system, designed as a CT imaging system, comprises a user interface that is configured to display a first screen after a user selects the short/long axis protocol. The user interface includes a protocol box and a plurality of views of the heart, such as an oblique long axis view including lines, a sagittal view including batch lines, and an axial view. The protocol box provides instructions for defining the short/long views of the heart. The protocol instructions include, among others, paging through the sagittal image to find an image with a mitral valve and apex of the heart, rotating a yellow line so that it joins these features. The user then selects "next" to bring up a plurality of batch lines to produce at least one 4-chamber long axis view.

US patent application 2006/0173268 A1 describes a system and method for interacting effectively with three-dimensional data such that a data acquisition system of an imaging system can be guided appropriately to gather relevant information from an object being imaged. The imaging system includes the data acquisition system for obtaining a three-dimensional image of the object that can be displayed on a computer workstation of a virtual user interface, and a processor coupled to the data acquisition system. The processor may be configured for receiving a user interface input based on interaction with the three-dimensional image, and for providing one or more scanning parameters to the data acquisition system based on the user interface input. These parameters may be used for further acquisition by the data acquisition system. The virtual user interface includes a computer workstation configured for displaying a three-dimensional image of the object.

In US patent application 2006/0072700 A1, an operating method for user interface for a computed tomography examination procedure is described in which a menu and a workflow list are merged on a computer screen. After inputting patient parameters into a parameter setting area, an operator determines a patient posture, i.e., a scanning program, from figures representing patient posture displayed in the parameter setting area of the display screen. After completing the scanning program selection, the operator selects the "scout view" option in the workflow list on the computer screen, and inputs scout view scanning parameters, such as scout view length, into the scanning parameter setting fields displayed in the parameter setting area. Then, the scout view is displayed in the scout view area on the computer screen. The operator determines a scanning plan from the displayed scout view, i.e., selecting an area to be scanned using a frame. Then, the operator sets scanning parameters, such as effective current (milliampere second, mAs), scanning time, slice thickness, dose and so on, in the scanning parameter setting fields displayed in said parameter setting area.

US patent application 2006/0193437 A1 describes a method and apparatus for controlling an imaging modality. The method includes steps of acquiring data specific to the examination object such as, for example, the age, the weight, the sex of the patient as well as further measures such as, for example, the overall size of the patient or of the body part to be examined are acquired. There then follows an automatic selection of an example raw data record from a number of example raw data records on the basis of the data specific to the examination object. An example image is then produced on the basis of the selected example raw data record by using a selected control parameter set. The produced image is then output to a user.

All the information that the operator must prescribe to the system can be input via a graphic user interface. The graphic user interface is subdivided into various sections. Located top left is a topogram output section, in which at least a portion of the topogram with the region of interest is displayed from which the profile of the patient in this area can be recognized. Located to the right thereof is a preview output section in which a current example image is displayed. Located below the topogram output section is a first input section, in which the operator can input, inter alia, the medical problem relevant to the examination and specific types of examination on the basis of which the examination protocol and/or the example raw data record are selected. The assistant input section and the selectable problems or types of examination are adapted in this case to the body region examined, which has been input previously when acquiring the patient data.

### SUMMARY OF THE INVENTION

It is therefore an object of the invention to alleviate the planning task of users when preparing an acquisition of image data of a particular anatomy.

According to the invention an apparatus and a method is proposed in accordance with one or more of the appended claims. In accordance with the invention also a computer program is proposed to have the apparatus of the invention operate in accordance with the method of the invention.

In a first aspect of the invention, an apparatus for acquiring diagnostic information is provided that comprises
- a data acquisition module for acquiring image data of at least a part of a person's anatomy,
- a planning module for defining with reference to a spatial position and orientation of an example anatomy at least one series of scanning steps to be executed with the data acquisition module, and
- a user interface that is configured to adjust imaging parameters to be used in a selected scanning step of the at least one series of scanning steps.

The user interface is configured to display for every step of a selected series of the at least one series of scanning steps predefined imaging parameters pertaining to the example anatomy.

Further, the user interface comprises a visual display unit having during use a first, exemplary window configured to show an image of the example anatomy of the selected scanning step, and a representation of the predefined imaging parameters of the selected scanning step, and a second, working window that is configured to show an image of an actual anatomy. The user interface is arranged to allow a user to adjust with reference to the image of the actual anatomy in said second, working window the imaging parameters to be used in the selected scanning step. The image of the actual anatomy is a three-dimensional survey volume of the person's anatomy.

This three dimensional survey volume may be acquired beforehand, or it may be acquired in real-time at the time when the examination is contemplated. The survey volume may also be obtained from another source than the apparatus of the invention. Amongst the imaging parameters that can be adjusted are an off-center with reference to a center point of a pre-acquired volume, an angulation, size, and foldover direction taking into account knowledge of the shape of a human body, and the general location of a region of interest.

The user can then be guided through the scanning procedure and plan the imaging parameters for each scanning step that will apply. This obviates the need for a user to be knowledgeable on a high-level with respect to the anatomy to be examined, and secures that also a less experienced user is able to plan and implement an entire examination of an actual anatomy.

The user can then simply compare the representation of the imaging parameters to be used in the selected scanning step pertaining to the actual anatomy, with the representation of the predefined imaging parameter as shown in the first, exemplary window, and decide whether the match between the two is sufficient for the purpose envisaged by the examination. This task can be further supported by arranging that the image of the actual anatomy shown in the second, working window corresponds in position and orientation to the image of the example anatomy shown in the first, exemplary window.

One of the beauties of the invention is that for any given examination a complete series of scanning steps can be comprised in the planning module. At times however it may be preferable that each selected scanning step of the at least one series of scanning steps in the planning module can be customized to meet site-specific preferences. In this way any particular examination institution, usually a hospital, can arrange to implement its own procedures.

It has proven to be very effective that the representation of the imaging parameters is indicated as a line displayed on the visual display unit, which line represents a line of sight of the data acquisition module. This means that the user of the apparatus only has to manipulate a similar line in the second, working window in order to define the imaging parameters pertaining to the selected scanning step.

It is then further beneficial to have a computer-mouse as input device, and that the line that is to represent the imaging parameters and that is to be displayed in the second, working window is drawable with a one click operation of the mouse.

### BRIEF DESCRIPTION OF THE DRAWINGS

The invention will hereinafter be further elucidated with reference to a drawing of an exemplary embodiment in accordance with the invention.

In the drawing:
Fig. 1 schematically shows the apparatus of the invention; and
Fig. 2 schematically shows images displayed during execution of the method of the invention on the visual display unit that forms part of the apparatus of the invention.

### DETAILED DESCRIPTION OF THE EMBODIMENTS

Wherever in the figures the same reference numerals are applied these numerals refer to the same parts.

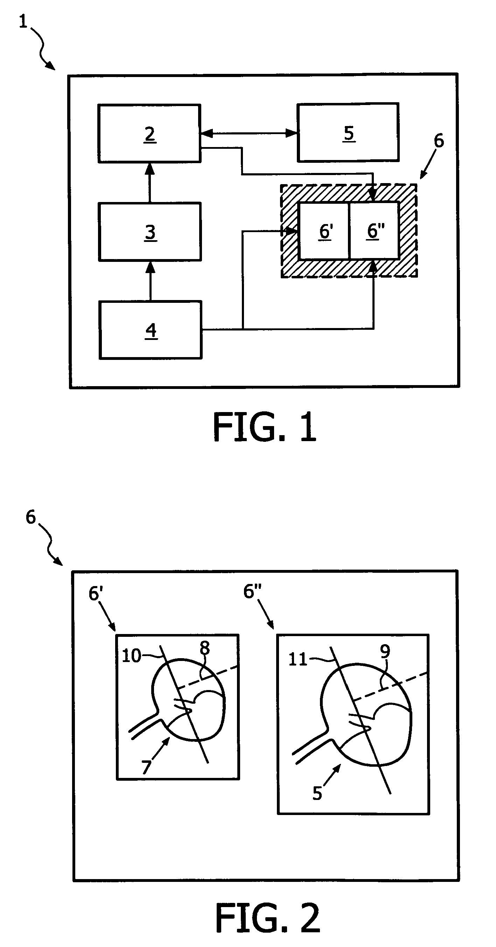

As mentioned in the introduction the data acquisition module to be used can be a magnetic resonance unit, a computer tomography unit, or any other type of commonly used data acquisition unit that is used for acquiring image data of a person's anatomy. In general such a data acquisition module is indicated with reference 2 in figure 1, and the anatomy to be examined is referred to with reference 5. The said data acquisition module 2 forms part of an apparatus 1 for acquiring diagnostic information pertaining to the anatomy 5, and is further provided with a planning module 3.

The planning module 3 has the purpose of defining with reference to a spatial position and orientation of an example anatomy a series of scanning steps to be performed when executing an examination, and which intends to have the data acquisition module 2 operate accordingly on the actual anatomy 5. When looking at the specific situation of having an MRI scanner perform a cardiac examination, this may for instance concern the object of acquiring an aortic valve view of the heart. A preferred planning path for the scanning operations to be executed for this purpose is to acquire subsequently the following views: transverse→right anterior oblique→nearly four chamber→short axis→four chamber→Basil short axis→left ventricular outflow tract→aortic valve. The user has to define the imaging parameters that are to be used in each of the scanning steps resulting in the just mentioned sequence of views, that collectively built the planning path that leads to the desired view of the aortic valve. It goes without saying that this is a complicated task if one cannot benefit from the apparatus and method according to the invention.

In the invention use is made of a user interface 4 to adjust the imaging parameters that are to be used in each of the scanning steps of the series of scanning steps, making up a complete examination card for acquiring the desired image data of the actual anatomy 5. For this purpose the user interface 4 displays for each single step in the selected series of scanning steps predefined scanning parameters pertaining to the example anatomy 7 in a window 6' of a visual display unit 6. Further the user interface 4 is arranged to have a user select the imaging parameters that are to be actually used in the prevailing scanning step that is next to be undertaken with reference to the actual anatomy 5, a three-dimensional survey volume of which is shown in the window 6" of the visual display unit 6. A preview showing an image visualizing the modified imaging parameters is shown in the window 6" . This image can be a simulation or can originate from processing preacquired data, and/or a real-time imaging using the data acquisition module, or even a different data acquisition module.

The manner in which the user interface 4 preferably lets the user select the desired imaging parameters applicable to any prevailing scanning step that applies for the collection of a desired (intermediate) view, for instance for the purpose of going from a transverse view of the heart to the right anterior oblique view, can be explained best with reference to figure 2.

Figure 2 shows the visual display unit 6 having a first, exemplary window 6' showing an image 7 of an example anatomy which can be subjected to a scanning step to go from the shown transverse view of the heart to the right anterior oblique view. For this purpose it is required to acquire image data according to a line of sight represented by the striped line 8 with reference to the example anatomy 7. It may be helpful to also show a helpline 10 perpendicular to the line 8. This can promote the accuracy of positioning an actual line of sight with reference to an actual anatomy 5. Accordingly in a second, working window 6" the visual display unit 6 shows the actual anatomy 5 and the scanning parameters represented by an adjustable line of sight embodied by the striped line 9 accompanied by a perpendicular helpline 11, the position and orientation of which lines the user can manipulate whilst having concurrently a view at the exemplary line of sight 8 and the perpendicular helpline 10 in the exemplary window 6'. In this manner the user is heavily assisted in drawing the line of sight 9 with reference to the actual anatomy 5 in order to realize an optimal match with the exemplary line of sight 8 in the exemplary window 6', which will meet the intended purpose of acquiring the image data pertaining to the right anterior oblique view of the heart. This type of operation also allows the user to tune the said line of sight 9 representing the scanning parameters of the prevailing scanning step in order to address the peculiarities of the actual anatomy 5.

For the acquisition of the other or further views, respectively the acquisition of the eventual view of the aortic valve, the method and apparatus of the invention are to be operated similarly in the scanning steps that are to be executed to obtain such other or further views.

It will be appreciated to those skilled in the art that many variations are feasible within the scope of the invention as defined by the appended claims and without departing the scope of protection as afforded by these claims.

## Claims

1. An apparatus (1) for acquiring diagnostic information comprising:
- a data acquisition module (2) for acquiring image data of at least a part of a person's anatomy (5),
- a planning module (2) for defining with reference to a spatial position and orientation of an example anatomy (7) at least one series of scanning steps to be executed with the data acquisition module (2),
- a user interface (4) configured to adjust imaging parameters to be used in a selected scanning step of the at least one series of scanning steps,
- wherein the user interface (4) is configured to display for every step of a selected series of the at least one series of scanning steps predefined imaging parameters (8) pertaining to the example anatomy (7),
wherein the user interface (4) comprises a visual display unit (6) having during use a first, exemplary window (6') configured to show an image of the example anatomy (7) of the selected scanning step, and a representation (8) of the predefined imaging parameters of the selected scanning step, and a second, working window (6") configured to show an image of an actual anatomy (5),
wherein the user interface (4) is arranged to allow a user to adjust with reference to the image of the actual anatomy (5) in said second, working window (6") the imaging parameters to be used in the selected scanning step, and
- wherein the image of the actual anatomy is a three-dimensional survey volume of the person's anatomy (5).

2. An apparatus according to claim 1, **characterized in that** the image of the actual anatomy (5) shown in the second, working window (6") corresponds in position and orientation to the image of the example anatomy (7) shown in the first, exemplary window (6').

3. An apparatus according to claim 1 or 2, **characterized in that** each of the at least one series of scanning steps in the planning module (2) can be customized to meet site-specific preferences.

4. An apparatus according to any one of claims 1-3, **characterized in that** the representation of the imaging parameters is indicated as a line (8, 9) displayed on the visual display unit (6), which line represents a line of sight of the data acquisition module (2).

5. An apparatus according to claim 4, having a mouse as input device, **characterized in that** the line (9) that is to represent the imaging parameters and that is to be displayed in the second, working window (6") is drawable with a one click operation of the mouse.

6. Method for acquiring diagnostic information comprising the steps:
- acquiring image data of at least a part of a person's anatomy (5),
- defining with reference to a spatial position and orientation of an example anatomy (7) at least one series of scanning steps to be executed with a data acquisition module (2),
- adjusting imaging parameters to be used in a selected scanning step of the at least one series of scanning steps,
**characterized by** the steps of
- displaying for every step of a selected series of the at least one series of scanning steps predefined imaging parameters (8) pertaining to the example anatomy (7),
- showing in a first, exemplary window (6') an image of the example anatomy (7) of the selected scanning step, and a representation (9) of the predefined imaging parameters of the selected scanning step,
- showing in a second, working window (6") an image of the actual anatomy (5),
- adjusting, by a user, with reference to the image of the actual anatomy (5) in said second, working window (6") the imaging parameters to be used in the selected scanning step,
wherein the image of the actual anatomy is a three-dimensional survey volume of the person's anatomy (5).

7. Method according to claim 6, **characterized in that** the image of the actual anatomy is shown in the second, working window (6") with a position and orientation that corresponds to the image of the example anatomy (5) shown in the first, exemplary window (6').

8. Method according to claim 6 or 7, **characterized in that** the representation of the imaging parameters of the selected scanning step is indicated as a line (8, 9) with reference to the shown anatomy (7, 5), which line represents a line of sight of the data acquisition module (2).

9. A computer program comprising instructions for causing the apparatus according to any one of claims 1-5 to carry out steps of the method according to any one of claims 6-8.

## Patentansprüche

1. Gerät (1) zum Erfassen von Diagnoseinformationen, das Folgendes umfasst:
- ein Datenerfassungsmodul (2) zum Erfassen von Bilddaten von mindestens einem Teil der Anatomie einer Person (5),
- ein Planungsmodul (2), um in Bezug auf eine räumliche Position und Ausrichtung einer Beispielanatomie (7) mindestens eine Reihe von Scanschritten zu definieren, die mit dem Datenerfassungsmodul (2) auszuführen sind,
- eine Benutzeroberfläche (4), die konfiguriert ist, um die in einem ausgewählten Scanschritt der mindestens einen Reihe von Scanschritten zu verwendenden Bildgebungsparameter zu justieren,
wobei die Benutzeroberfläche (4) konfiguriert ist, um für jeden Schritt einer ausgewählten Reihe der mindestens einen Reihe von Scanschritten vordefinierte Bildgebungsparameter (8) anzuzeigen, die zu der Beispielanatomie (7) gehören,
wobei die Benutzeroberfläche (4) eine visuelle Anzeigeeinheit (6) umfasst, das während des Betriebs ein erstes, beispielhaftes Fenster (6') aufweist, welches konfiguriert ist, um ein Bild der Beispielanatomie (7) des ausgewählten Scanschritts und eine Darstellung (8) der vordefinierten Bildgebungsparameter des ausgewählten Scanschritts anzuzeigen, sowie ein zweites Arbeitsfenster (6") aufweist, das konfiguriert ist, um ein Bild einer tatsächlichen Anatomie (5) anzuzeigen,
wobei die Benutzeroberfläche (4) dafür ausgelegt ist, dem Benutzer zu ermöglichen, die in dem ausgewählten Scanschritt zu verwendenden Bildgebungsparameter unter Bezugnahme auf das Bild der tatsächlichen Anatomie (5) in dem genannten zweiten Arbeitsfenster (6") einzustellen, und
- wobei das Bild der tatsächlichen Anatomie ein dreidimensionales Übersichtsvolumen der Anatomie einer Person (5) ist.

2. Gerät nach Anspruch 1, **dadurch gekennzeichnet, dass** das Bild der tatsächlichen Anatomie (5), das in dem zweiten Arbeitsfenster (6") angezeigt wird, in der Position und Ausrichtung mit dem Bild der Beispielanatomie (7) aus dem ersten, beispielhaften Fenster (6') übereinstimmt.

3. Gerät nach Anspruch 1 oder 2, **dadurch gekennzeichnet, dass** jeder der mindestens einen Reihe von Abtastschritten in dem Planungsmodul (2) an die ortsspezifischen Präferenzen angepasst werden kann.

4. Gerät nach einem der Ansprüche 1 bis 3, **dadurch gekennzeichnet, dass** die Darstellung der Bildparameter als eine auf der visuellen Anzeigeeinheit (6) angezeigte Linie (8, 9) angegeben wird, wobei die Linie eine Sichtlinie des Datenerfassungsmoduls (2) darstellt.

5. Gerät nach Anspruch 4, mit einer Maus als Eingabevorrichtung, **dadurch gekennzeichnet, dass** die Linie (9), die die Bildgebungsparameter darstellen soll und die in dem zweiten Arbeitsfenster (6") angezeigt werden soll, mit einer Klickbetätigung der Maus gezogen werden kann.

6. Verfahren zum Erfassen von Diagnoseinformationen, das die folgenden Schritte umfasst:
- Erfassen von Bilddaten von mindestens einem Teil der Anatomie einer Person (5),
- in Bezug auf eine räumliche Position und Ausrichtung einer Beispielanatomie (7) Definieren von mindestens einer Reihe von Scanschritten, die mit dem Datenerfassungsmodul (2) auszuführen sind,
- Justieren der Bildgebungsparameter, die in einem ausgewählten Scanschritt der mindestens einen Reihe von Scanschritten zu verwenden sind,
**gekennzeichnet durch** die folgenden Schritte:
- für jeden Schritt einer ausgewählten Reihe der mindestens einen Reihe von Scanschritten Anzeigen von vordefinierten Bildgebungsparametern (8), die zu der Beispielanatomie (7) gehören,
- in einem ersten beispielhaften Fenster (6') Anzeigen eines Bilds der Beispielanatomie (7) des ausgewählten Scanschritts und einer Darstellung (9) der vordefinierten Bildgebungsparameter des ausgewählten Scanschritts anzuzeigen;
- in einem zweiten Arbeitsfenster (6") Anzeigen eines Bilds der tatsächlichen Anatomie (5),
- Einstellen seitens des Benutzers der in dem ausgewählten Scanschritt zu verwendenden Bildgebungsparameter unter Bezugnahme auf das Bild der tatsächlichen Anatomie (5) in dem genannten zweiten Arbeitsfenster (6"), und
wobei das Bild der tatsächlichen Anatomie ein dreidimensionales Übersichtsvolumen der Anatomie einer Person (5) ist.

7. Verfahren nach Anspruch 6, **dadurch gekennzeichnet, dass** das Bild der eigentlichen Anatomie, das in dem zweiten Arbeitsfenster (6") angezeigt wird, in der Position und Ausrichtung mit dem Bild der Beispielanatomie (5) aus dem ersten, beispielhaften Fenster (6') übereinstimmt.

8. Verfahren nach Anspruch 6 oder 7, **dadurch gekennzeichnet, dass** die Darstellung der Bildparameter des ausgewählten Scanschritts als eine Linie (8, 9) in Bezug auf die angezeigte Anatomie (7, 5) angegeben wird, wobei die Linie eine Sichtlinie des Datenerfassungsmoduls (2) darstellt.

9. Computerprogramm mit Anweisungen zum Veranlassen des Geräts nach einem der Ansprüche 1 bis 5, die Schritte des Verfahrens nach einem der Schritte 6 bis 8 auszuführen.

## Revendications

1. Appareil (1) pour acquérir des informations diagnostiques, comprenant :
- un module d'acquisition de données (2) pour acquérir des données d'image d'au moins une partie de l'anatomie d'une personne (5),
- un module de planification (2) pour définir, par rapport à une position et une orientation spatiales d'une anatomie illustrative (7), au moins une série d'étapes de balayage destinées à être exécutées avec le module d'acquisition de données (2),
- une interface utilisateur (4) configurée pour ajuster des paramètres d'imagerie destinés à être utilisés dans une étape de balayage sélectionnée parmi l'au moins une série d'étapes de balayage,
dans lequel l'interface utilisateur (4) est configurée pour afficher, pour chaque étape d'une série sélectionnée parmi l'au moins une série d'étapes de balayage, des paramètres d'imagerie prédéfinis (8) se rapportant à l'anatomie illustrative (7),
dans lequel l'interface utilisateur (4) comprend une unité d'affichage visuel (6) comportant, durant l'utilisation, une première fenêtre illustrative (6') configurée pour présenter une image de l'anatomie illustrative (7) de l'étape de balayage sélectionnée, et une représentation (8) des paramètres d'imagerie prédéfinis de l'étape de balayage sélectionnée, et une seconde fenêtre active (6") configurée pour présenter une image d'une anatomie réelle (5),
dans lequel l'interface utilisateur (4) est agencée pour permettre à un utilisateur d'ajuster, par rapport à l'image de l'anatomie réelle (5) dans ladite seconde fenêtre active (6"), les paramètres d'imagerie destinés à être utilisés dans l'étape de balayage sélectionnée, et
dans lequel l'image de l'anatomie réelle est un volume d'inspection tridimensionnel de l'anatomie de la personne (5).

2. Appareil selon la revendication 1, **caractérisé en ce que** l'image de l'anatomie réelle (5) présentée dans la seconde fenêtre active (6") correspond en position et orientation à l'image de l'anatomie illustrative (7) présentée dans la première fenêtre illustrative (6').

3. Appareil selon la revendication 1 ou 2, **caractérisé en ce que** chacune parmi l'au moins une série d'étapes de balayage dans le module de planification (2) peut être adaptée pour satisfaire à des préférences spécifiques au site.

4. Appareil selon l'une quelconque des revendications 1 à 3, **caractérisé en ce que** la représentation des paramètres d'imagerie est indiquée sous forme de ligne (8, 9) affichée sur l'unité d'affichage visuel (6), laquelle ligne représente une ligne de visée du module d'acquisition de données (2).

5. Appareil selon la revendication 4, comportant une souris en tant que dispositif d'entrée, **caractérisé en ce que** la ligne (9) qui doit représenter les paramètres d'imagerie et qui doit être affichée dans la seconde fenêtre active (6") peut être tirée avec une opération à un clic de la souris.

6. Procédé pour acquérir des informations diagnostiques comprenant les étapes :
- l'acquisition de données d'image d'au moins une partie de l'anatomie (5) d'une personne,
- la définition par rapport à une position et une orientation spatiales d'une anatomie illustrative (7) d'au moins une série d'étapes de balayage destinées à être exécutées avec un module d'acquisition de données (2),
- l'ajustement de paramètres d'imagerie destinés à être utilisés dans une étape de balayage sélectionnée parmi l'au moins une série d'étapes de balayage,
**caractérisé par** les étapes de :
- l'affichage, pour chaque étape d'une série sélectionnée parmi l'au moins une série d'étapes de balayage, de paramètres d'imagerie prédéfinis (8) se rapportant à l'anatomie illustrative (7),
- la présentation, dans une première fenêtre illustrative (6'), d'une image de l'anatomie illustrative (7) de l'étape de balayage sélectionnée, et d'une représentation (9) des paramètres d'imagerie prédéfinis de l'étape de balayage sélectionnée,
- la présentation, dans une seconde fenêtre active (6"), d'une image de l'anatomie réelle (5),
- l'ajustement, par un utilisateur, par rapport à l'image de l'anatomie réelle (5) dans ladite seconde fenêtre active (6"), des paramètres d'imagerie devant être utilisés dans l'étape de balayage sélectionnée,
dans lequel l'image de l'anatomie réelle est un volume d'inspection tridimensionnel de l'anatomie de la personne (5).

7. Procédé selon la revendication 6, **caractérisé en ce que** l'image de l'anatomie réelle est présentée dans la seconde fenêtre active (6") avec une position et une orientation qui correspondent à l'image de l'anatomie illustrative (5) présentée dans la première fenêtre illustrative (6').

8. Procédé selon la revendication 6 ou 7, **caractérisé en ce que** la représentation des paramètres d'imagerie de l'étape de balayage sélectionnée est indiquée sous forme de ligne (8, 9) par rapport à l'anatomie présentée (7, 5), laquelle ligne représente une line de visée du module d'acquisition de données (2).

9. Programme d'ordinateur comprenant des instructions pour faire en sorte que l'appareil selon l'une quelconque des revendications 1 à 5 réalise les étapes du procédé selon l'une quelconque des revendications 6 à 8.
